# DEMANDE DE BREVET EUROPEEN

(11) **EP 0 581 667 A1**
(43) Date de publication de la demande: **02.02.1994**
(21) Numéro de dépôt: 93401939.9
(22) Date de dépôt: 27.07.1993
(51) Int. Cl.: A61F 2/40

(54) **Prothèse de glène vissée**

(30) Priorité: 29.07.1992 FR 9209343
(71) Demandeur: SCIENCE ET MEDECINE SA, F-75012 Paris (FR)
(72) Inventeur: Breard, Francis Henri, F-75014 Paris (FR)

(57) **Abrégé**

La présente invention concerne une prothèse de glène destinée à être vissée dans l'omoplate caractérisée en ce qu'elle comprend une embase filetée dans laquelle viennent s'im- pacter un bouclier puis un coussin, ce dernier s'interposant entre le bouclier et la prothèse humérale.

## Description

La présente invention concerne un perfectionnement de prothèse totale d'épaule, et plus particulièrement un élément glénoïde destiné à être fixé à la cavité glénoïde de l'omoplate et à recevoir la tête de l'implant huméral.

La jonction os/prothèse est une zone de fragilité. Or, il est indispensable que cette jonction soit la plus solide possible afin de permettre le mouvement de l'articulation et éviter les douleurs en cas de mauvaises fixations. La technique d'implantation des prothèses d'épaule s'est développée avec l'apparition des prothèses cimentées de Neer, mais les descellements de la glène prothétique sont fréquents.

Il est connu à l'heure actuelle d'ancrer l'élément glénoïde à l'aide d'un ciment dans un espace préalablement préparé par résection osseuse dans l'omoplate au niveau de la cavité glénoïde naturelle défectueuse. Mais ce principe présente des inconvénients : en cas d'erreur d'implantation, tout rattrapage ultérieur est problématique, voire impossible, l'ancrage de l'élément dans l'omoplate étant souvent délicat à réaliser, notamment quand le tissu osseux est fortement détérioré et présente des parties spongieuses cassantes.

De plus, le ciment présente des caractéristiques mécaniques éloignées de celles de l'os et difficilement contrôlables dans le temps, des risques per- opératoire dûs au choc thermique résultant de sa polymérisation, l'apparition d'un liseré parfois évolutif à l'interface os/ciment visible et inexpliqué sur les radiographies.

On connaît également une technique consistant à assembler l'élément glénoïde à l'omoplate par des vis rapportées. Mais ce principe présente le désavantage d'une mise en place peu aisée et approximative, ainsi qu'une stabilité peu fiable dans le temps par rupture des vis.

La présente invention se propose de remédier à ces inconvénients et, pour ce faire, elle a pour objet une prothèse de glène de type nouveau destinée à être vissée dans l'omoplate, caractérisée en ce qu'elle comprend une embase filetée dans laquelle viennent s'impacter un bouclier puis un coussin, ce dernier s'interposant entre le bouclier et la prothèse humérale.

Cette prothèse offre une possibilité de reprise (en cas d'erreur d'implantation), soit sur la surface portante en conservant intacte la partie vissée profondément ancrée dans la cavité glénoïde de l'omoplate, soit par ablation totale se faisant par un simple dévissage de l'embase, ce qui a pour intérêt de ne pas détériorer l'os.

Dans sa forme la plus simple, l'embase filetée est munie d'une partie platine multipercée et d'un plot central creusé d'un multipans femelle.

Avantageusement, le plot central est une vis à gros filet cylindrique ou conique. Plusieurs types de vis peuvent cependant être utilisés, à simples ou multiples filets, avec ou sans rainure auto-taraudeuse.

Selon un mode préférentiel de réalisation, le plot est perforé en son centre afin de pouvoir s'enfiler sur la vis broche. L'embase filetée est de préférence en titane pur. Elle permet ainsi une bonne ostéointégra- tion renforcée par l'action mécanique de la vis et donne l'assurance d'une immobilisation totale et durable.

Le bouclier est porteur d'un multipans mâle et de plots d'ancrage.

Le bouclier et l'embase s'assemblent donc par le multipans, ce qui permet de les encastrer parfaitement l'un dans l'autre de façon à avoir un ensemble statique entre implant et os, le multipans mâle assurant un positionnement du bouclier de forme anatomique par rapport à la glène.

Le multipans femelle comporte de préférence une bague de verrouillage, par exemple en polyéthylène haute densité. De même, le multipans male comporte une cannelure verticale.

Cependant, il est certain que le multipans mentionné dans cette description peut être remplacé par toute autre forme (conique ou totalement cylindrique dans le cas d'une demande de liberté totale en rotation).

Selon une autre caractéristique de l'invention, les plots du bouclier traversent la platine de l'embase et se plantent dans l'os, ce qui forme un système anti- rotatif. Les plots peuvent être de forme cylindrique, avec ou sans gorges, tronconiques, pyramidaux de sections triangulaires ou carrées ou toute forme pouvant être impactée facilement et permettant une os- téointégration aisée.

Leur guidage dans l'embase filetée se fait automatiquement par alignement du multipans et des trous.

Une fois ces plots implantés dans l'os, le coussin préablement ou postérieurement fixé peut recevoir la tête de la prothèse humérale, ce coussin pouvant avoir des épaisseurs différentes, des rayons de surface portante différents pouvant s'adapter à toutes les prothèses et étant de préférence en polyéthylène haute densité.

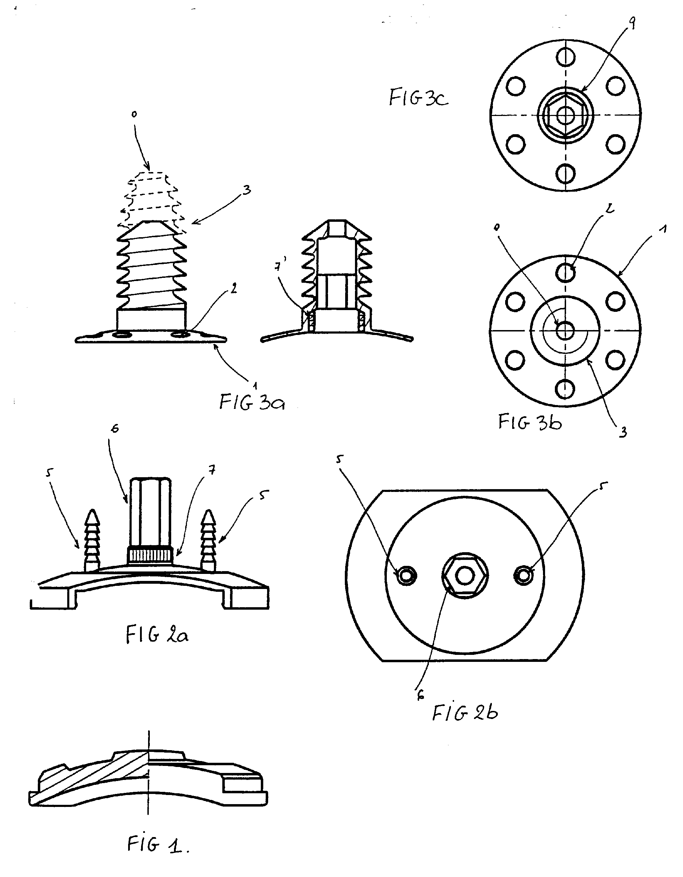

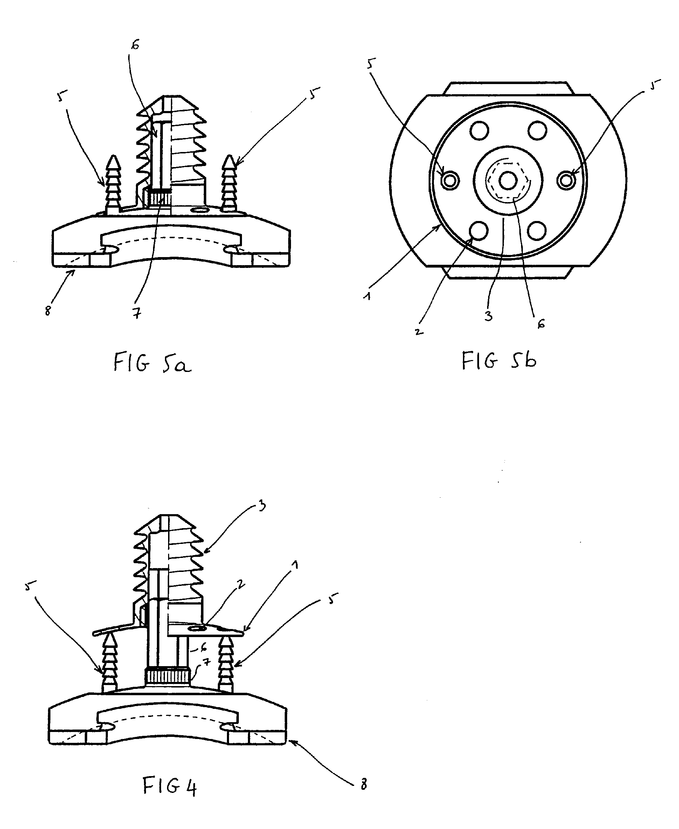

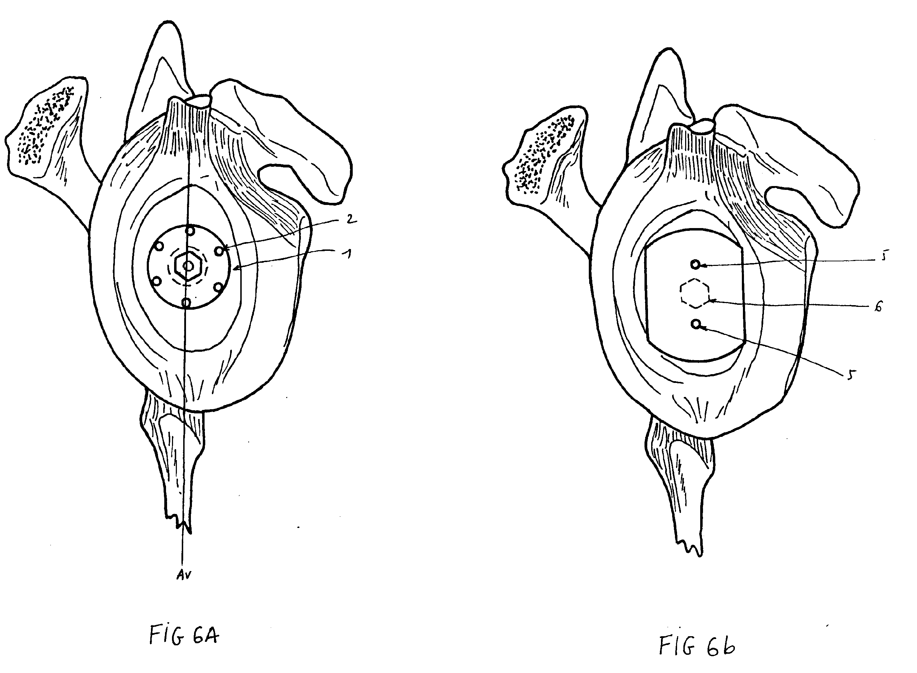

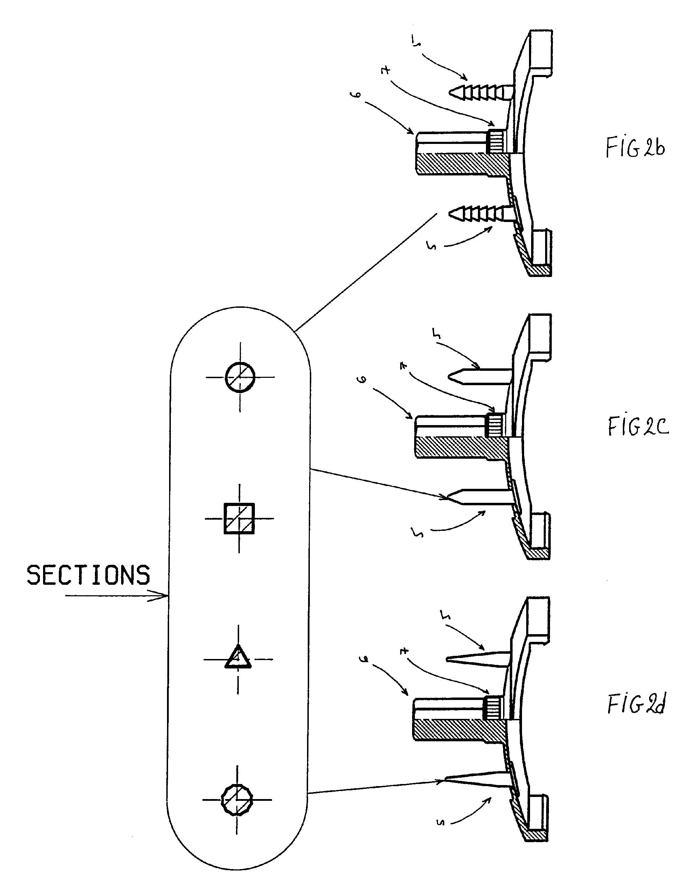

D'autres caractéristiques du dispositif glénoïde vont maintenant être décrites, mais sans caractère limitatif, en référence aux dessins annexés sur lesquels :
. La figure 1 montre en vue de face le coussin.
. Les figures 2a et 2b représentent respectivement en vue de face en coupe et en vue de dessus le bouclier.
. Les figures 2c et 2d montrent ce même bouclier en vue de face avec des plots de formes différentes.
. Les figures 3a, 3b et 3c illustrent respectivement en vue de face et en vue de dessus, de dessous l'embase filetée.
. La figure 4 montre le bouclier venant s'encastrer dans l'embase;
e Les figures 5a et 5b sont respectivement une vue de face et de dessus du dispositif glénoïde, objet de l'invention.
e Les figures 6a et 6b représentent respectivement l'emplacement de l'embase et du bouclier dans la cavité glénoïde .

La figure 3a représente une embase filetée et perforée (0) comportant une platine circulaire (1) percée (2) et une vis à gros filet (3). Le vissage de l'embase doit être effectué "à fond" pour que la platine percée se retrouve au contact de l'os comme le montre la figure 6a. Cette mise en place s'effectue à l'aide de la broche-guide et d'un ancillaire simple (fraise et taraud) et adapté à la préhension des éléments prothétiques. La broche est plantée dans le centre de la cavité glénoïde de l'omoplate. Cette broche de 3 à 6 cm servira de guide permanent j'usqu'à la fin de l'ancrage de l'embase filetée dans l'os. Implantée à une de ses extrémités dans l'os et à l'autre dans l'embase perforée (0), elle permet une précision optimale de direction d'insertion. Une fois implantée, l'embase se retrouve dans une position aléatoire, et il suffit d'effectuer une rotation minime dans le sens du serrage pour trouver un couple de trous, diamétralement opposés se confondant avec l'axe vertical (AV).

Le bouclier et l'embase filetée s'assemblent par un six pans comme le montre la figure 4, ce qui permet de les positionner l'un par rapport à l'autre, les plots du bouclier (5) venant s'encastrer dans l'os préalablement guidé ou rendu complètement solidaire de l'embase filetée par déformation des trous de passage apparents sur celle-ci (7).

Le bouclier représenté par les figures 2a et 2b constitué d'un six pans mâle (6) possède en sa jonction une partie cannelée droite (7) venant s'encastrer pendant l'impaction dans la bague de verrouillage de l'embase filetée (7'). Le couple bague/cannelure permet ainsi un arrêt primaire de translation et de rotation.

La rotation du bouclier dans l'embase filetée, le dévissage de l'ensemble et les micro-mouvements sont supprimés par la présence du six pans et des plots.

Tous les degrés de liberté étant supprimés, la glène se trouve donc immobilisée, la prothèse humérale vient se positionner dans le coussin (8) .

## Revendications

1. Prothèse de glène destinée à être vissée dans l'omoplate, comportant une embase filetée (Fig 3a) dans laquelle viennent s'impacter un bouclier (Fig 2a), puis un coussin (Fig 1), caractérisée en ce que l'embase filetée est constituée d'une platine (1) multipercée (2), d'un plot central (3) creusé d'un multipans femelle, en ce que le bouclier (Fig 2a) est muni d'un multipans male (6) et de plots d'ancrage (5).

2. Prothèse selon la revendication 1, caractérisée en ce que le plot central (3) de l'embase (Fig 3a) est une vis à gros filet cylindrique ou conique, à simple ou multi filets, avec ou sans rainure auto-taraudeuse.

3. Prothèse selon les revendications 1 et 2, caractérisée en ce que l'embase (Fig 3a) filetée est perforée (0) en son centre.

4. Prothèse selon les revendications 1 à 3, caractérisée en ce que les plots (5) du bouclier sont de forme cylindrique avec ou sans gorges, tronconi- i-ques, pyramidaux ou toute forme pouvant être impactée facilement et permettant une ostéointé- gration aisée.

5. Prothèse selon les revendications 1 à 4, caractérisée en ce que le bouclier (Fig 2a) et l'embase filetée (Fig 3a) s'assemblent par toute forme permettant un indexage multiple ainsi que conique ou totalement cylindrique.

6. Prothèse selon les revendications 1 à 5, caractérisée en ce que le guidage des plots (5) du bouclier (Fig 2a) dans l'embase filetée (Fig 3a) se fait automatiquement par alignement du multipans et des trous.

7. Prothèse selon les revendications 1 à 6, caractérisée en ce que les plots (5) du bouclier (Fig 2a) traversent la platine (1) de l'embase (Fig 3a) et se plantent dans l'os.

8. Prothèse selon les revendications 1 à 7, caractérisée en ce que le multipans femelle est munie en sa partie interne d'une bague de verrouillage (7').

9. Prothèse selon les revendications 1 à 8, caractérisée en ce que le multipans mâle (6) du bouclier (Fig 2a) possède, en sa base, une partie cannelée droite (7) venant s'encastrer, pendant l'impaction, dans la bague (7') de l'embase filetée.

10. Prothèse selon les revendications 1 à 9, caractérisée en ce que le coussin (Fig 1) peut avoir des épaisseurs différentes, des rayons de surface portante différents pouvant s'adapter à toutes les prothèses.

11. Prothèse selon les revendications 1 à 10, caractérisée en ce que le coussin (Fig 1) et la bague de verrouillage (7') sont en polyéthylène haute densité.
